Europäisches Patentamt

European Patent Office

Office européen des brevets

(11) Veröffentlichungsnummer: **0 431 385 A1**

## (12) EUROPÄISCHE PATENTANMELDUNG

(21) Anmeldenummer: **90122114.3**

(22) Anmeldetag: **20.11.90**

(51) Int. Cl.5: **A01N 1/02, G01N 33/68**

(30) Priorität: **24.11.89 DE 3938907**

(43) Veröffentlichungstag der Anmeldung:
**12.06.91 Patentblatt 91/24**

(84) Benannte Vertragsstaaten:
**AT BE CH DE ES FR GB IT LI LU NL SE**

(71) Anmelder: **BEHRINGWERKE
Aktiengesellschaft
Postfach 1140
W-3550 Marburg 1(DE)**

(72) Erfinder: **Fickenscher, Karl, Dr.
Sonnenweg 8
W-3550 Marburg(DE)**

(74) Vertreter: **Klein, Otto, Dr. et al
Hoechst AG Zentrale Patentabteilung
Postfach 80 03 20
W-6230 Frankfurt am Main 80(DE)**

(54) Mittel zum Lagern und Suspendieren von Zellen, insbesondere Erythrozyten.

(57) Die Erfindung betrifft ein Mittel zum Konservieren, Lagern und Suspendieren von Zellen, insbesondere Erythrozyten, welches, neben anderen dem Fachmann bekannten Substanzen wie z.B. Elekrolyten und Zuckern, einen Chelatbildner für mehrwertige Metallionen enthält.

EP 0 431 385 A1

## MITTEL ZUM LAGERN UND SUSPENDIEREN VON ZELLEN, INSBESONDERE ERYTHROZYTEN

Die Erfindung betrifft ein Mittel zum Konservieren, Lagern und Suspendieren von Zellen, insbesondere Erythrozyten, welches, neben anderen dem Fachmann bekannten Substanzen wie z.B. Elekrolyten und Zuckern, einen Chelatbildner für mehrwertige Metallionen enthält.

Zur Herstellung von Reagenzien zur Bestimmung von bestimmten Parametern (z.B. Komplementbestimmung) werden aus antikoaguliertem Vollblut isolierte und stabilisierte Erythrozyten benötigt.

Im Rahmen von Blutkomponententherapien werden aus Vollblut (Blutkonserven) durch Plasmaabtrennung Erythrozytenkonzentrate hergestellt. Diese Konzentrate finden heute vielfache therapeutische Anwendung, z. B. bei vorliegender Anämie bzw. wenn die Indikation Erythrozytenzufuhr besteht. Um die Erythrozytenkonzentrate auf eine physiologisch verträgliche Viskosität einzustellen, muß eine Verdünnungslösung zugesetzt werden, mit der auch, für die Erhöhung der Lagerungsfähigkeit notwendige, Substanzen zugeführt werden können.

Zum Suspendieren und Lagern von Zellen, insbesondere Erythrozyten, eignen sich verschiedene Mittel. So wird z.B. in der WO 81/02239 eine Lösung beschrieben, die Adenin, Mannitol, Glukose oder Fruktose und Natriumchlorid enthält. In der WO 86/00809 wird eine Lösung beschrieben, die Adenin, Mannitol, Glukose, Kaliumcitrat und Ammoniumchlorid bzw. Ammoniumacecat enthält. In der WO 87/04072 wird eine Lösung beschrieben, die L-Aminosäuren oder deren Derivate, Fettsäuren oder deren Derivate, Intermediärprodukte der Glykolyse wie Phosphoenolpyruvat und Analoga davon, Nukleoside wie Adenosin oder Guanosin, Adenosinmonophosphat und Abkömmlinge davon, Ammonium, Glycogen, Acetyl-CoA und Abkömmlinge, Allantoin, 4-Ethyloxaloacetat, Phenylethylbiguanid und Analoga, Quercetin, Kobaltsulfat, Nickelsulfat, Magnesiumchlorid, Manganchlorid sowie diverse Inhibitoren der Di-Phospho-Glycerat-Phosphatase als Zusätze zu den Standardkomponenten Antikoagulans, Zuckeralkohol, Glukose und Natriumchlorid enthalten kann. In der WO 89/02274 wird eine Lösung beschrieben, die Glukose, Natriumchlorid, Kaliumchlorid, Calciumchlorid, Magnesiumsulfat, Natriumphosphat, Natriumcitrat und Natriumbicarbonat enthält. In der US-PS 4 267 269 wird eine Lösung beschrieben, die neben Adenin den Zuckeralkohol Mannit, Glukose oder Fruktose und Natriumchlorid enthält. Die US-PS 4 356 172 beschreibt eine Lösung die Adenin oder Inosin, Saccharose oder Laktose, sowie Zitronensäure oder ein Salz davon enthält. Die US-PS 4 704 352 beschreibt eine Lösung, die zur Stabilisierung von Blutzellen einen pH von unter 7.0 aufweist, sowie Adenin, Mannit, Dextrose, Natriumchlorid und L-Ascorbat-2-Phosphat enthält. Die US-PS 4 769 318 beschreibt die Stabilisierung und Aktivierung von Blut vor der Transfusion mittels eines Derivats von Phosphoenolpyruvat sowie Adenin, einem Saccharid (Saccharose, Maltose, Galaktose oder Mannitol), Zitronensäure bzw. deren Natriumsalz (auch EP 0 275 198). Die EP 0 099 315 beschreibt eine Phosphat-gepufferte Lösung die Adenin, Glukose, Saccharose und Natriumchlorid enthält. Die EP 0 100 419 beschreibt eine Phosphatgepufferte Lösung die Adenin und/oder Guanosin, einen Zuckeralkohol (Sorbit oder Xylit), Glukose oder Fruktose, Natriumchlorid sowie Kolloide enthält. Die EP 0 301 250 beschreibt eine Phosphat-gepufferte Lösung die Adenin und/oder Guanosin, einen Zuckeralkohol (Sorbit, Mannit oder Xylit), Glukose oder Fruktose, Natriumchlorid sowie Kolloide enthält. (Siehe auch DE-A-37 22 984). Die DE 3 220 232 beschreibt ein Verfahren und eine Lösung zur Stabilisierung von Blutplättchen durch Einsatz von Jodacetamid, einer Iminoessigsäure und einem bakteriostatischem Agens. Die DE-A-3 225 408 beschreibt eine Lösung die Adenin oder Guanosin, einen Zuckeralkohol (Sorbit oder Xylit), Glukose oder Fruktose, enthält. Ferner beschreiben Heaton et al. (in British Journal of Haematology 57, 467-478, 1984) eine Lösung (ADSOL(R)), im wesentlichen bestehend aus Adenin, Mannitol, Dextrose und Natriumchlorid zur Stabilisierung von Erythrozyten. Ebenfalls bekannt ist die stabilisierende Wirkung des humantoxischen Weichmachers Diethylhexylphthalat (Horowitz B. et al. Vox Sang. 48, 150-155, 1985).

Durch Zugabe solcher Suspensionslösungen zu Erythrozytenkonzentraten wurde zwar die Überlebensrate und damit die Lagerungsfähigkeit von den Erythrozyten verbessert, aber diese Verbesserungen waren zu gering, um den hohen Anforderungen, z.B. an die Mindestlaufzeiten eines Diagnostikums zu genügen. Es war auch nicht möglich, damit ein gebrauchsfertiges Reagenz herzustellen, da sich die eingestellte Erythrozytenkonzentration sowie die Eigenschaften der Erythrozyten während der Lagerung veränderten.

Der Erfindung lag daher die Aufgabe zugrunde, die Überlebensrate und damit die Lagerungsfähigkeit von Zellen bzw. die Hämolyserate von Erythrozyten zu verbessern.

Die vorliegende Erfindung betrifft nun Mittel zum Suspendieren, Konservieren und Lagern von biologisch aktiven Zellen oder Zellbestandteilen, insbesondere Erythrozyten, wobei das Mittel neben anderen, dem Fachmann bekannten Substanzen wie z. B. Antikoagulantien, Elektrolyten, Zuckern und Zuckeralkoholen, einen Chelatbildner für mehrwertige Metallionen in einer gepufferten, wässrigen Lösung enthält. Überraschenderweise wurde gefunden, daß Zellen, insbesondere Erythrozyten eine wesentlich erhöhte Überlebensrate und stark verringerte Hämolyserate zeigen, wenn sie in einer solchen Lösung gelagert werden.

Chelatbildner für mehrwertige Metallionen sind dem Fachmann bekannte Verbindungen. Bevorzugterweise werden Chelatbildner für zweiwertige Metallionen verwendet, besonders bevorzugt ist die Verwendung von Ethylendiamin-Tetraacetat (EDTA), Ethylenglycol-bis-Aminoethylether-Tetraacetat (EGTA) oder Oxalsäure, ganz besonders bevorzugt verwendet wird EGTA.

Die Chelatbildner werden in Konzentrationen von 0 bis 100 mmol/l, bevorzugterweise von 0.1 bis 50 mmol/l und besonders bevorzugterweise von 2 - 10 mmol/l eingesetzt.

Als Elektrolyten kommen insbesonders Natrium-, Kalium-und Magnesiumsalze in der Form der Chloride, Sulfate oder Citrate in Betracht.

Als Zucker werden bevorzugt verwendet Saccharose und Fruktose, als Zuckeralkohol Mannit, Glukose und Sorbit.

Als weitere Zusatzstoffe kommen u. a. in Betracht Kolloide, wobei Gelatine und Polygelin bevorzugt sind.

Als Antikoagulantien bevorzugt sind Heparin und Citrat. Die Stabilität kann desweiteren durch die Zugabe eines für die Zellen unschädlichen Antibiotikums wie z. B. Kathon DP$^{(R)}$ (Röhm & Haas) oder Chloramphenicol verbessert werden.

Das erfindungsgemäße Mittel kann darüberhinaus weitere, dem Fachmann bekannte, Substanzen aus der Klasse der Nucleoside (z. B. Adenosin oder Guanosin), aus der Klasse der Purinbasen (z. B. Adenin) oder Intermediärprodukte und Substrate der Glykolyse enthalten.

Andere Zusätze, die in den bekannten Mitteln verwendet werden, können auch mit dem erfindungsgemäßen Mittel kombiniert werden.

Die günstigen Eigenschaften einer derartigen Lösung lassen sich noch verbessern, wenn Adenosintriphosphat (ATP) zugegeben wird und/oder als zweite Puffersubstanz ein Natriumsalz der Phosphorsäure eingesetzt wird.

Puffersubstanzen sind alle physiologisch verträglichen Puffersubstanzen, bevorzugterweise wird HEPES oder ein wasserlösliches Phosphat verwendet.

Ganz besonders bevorzugt sind die Mittel gemäß Beispiel 2 und 3.

Eine typische verwendungsfähige Lösung setzt sich beispielsweise wie folgt zusammen: Pro Liter Lösung, gelöst in Aqua dest. 0 bis 100 mmol Glukose, 20 bis 200 mmol Natriumchlorid, 0 bis 50 mmol Kaliumchlorid, 0 bis 10 mmol Magnesiumchlorid, 2 bis 100 mmol HEPES, 0 bis 100 mmol Na-Phosphat, 0.1 bis 50 mmol Ethylendiamin-Tetraacetat (EDTA), Ethylenglycol-bis-Aminoethylether-Tetraacetat (EGTA) oder Oxalessigsäure, vorzugsweise EGTA, 0 bis 20 000 Einheiten Heparin, 0 bis 5 g Gelatine, 0 bis 50 mmol ATP und 0 bis 1 g Kathon oder Chloramphenicol, pH 5.0 bis 9.0, vorzugsweise 6.5 bis 7.5 und ganz besonders bevorzugt 6.8 bei Verwendung von Kathon und 7.4 bei Verwendung von Chloramphenicol. Bevorzugterweise ist die Lösung so zusammengesetzt, daß sie in etwa isotonisch ist.

Zur Anwendung dieser Lösungen wird üblicherweise so verfahren, daß ein mit Citrat oder Heparin stabilisiertes Vollblut menschlichen oder tierischen Ursprungs herge stellt wird. Das Vollblut wird zentrifugiert und das Plasma einschließlich der Grenzschicht zu den Erythrozyten entfernt. Anschließend werden die Erythrozyten zweimal in der Pufferlösung ohne ATP gewaschen. Die gewaschenen Erythrozyten werden anschließend mit dem vollständigen Puffer auf die gewünschte Konzentration gebracht und bei 2-8 °C gelagert oder sofort verwendet.

Die folgenden Beispiele dienen zur Erläuterung der Erfindung, schränken sie aber in keiner Weise ein.

Beispiel 1

Herstellung einer Erythrozytensuspension für diagnostische Zwecke

300 ml Blut werden unter sterilen Bedingungen aus einem Schaf in eine Citratvorlage als Antikoagulans entnommen. Zur Abtrennung des Plasmas wird das Vollblut bei 1000 x g für 15 min zentrifugiert. Das Plasma wird einschließlich der Grenzschicht zum Zellkuchen abgesaugt und die Erythrozyten im gleichen Volumen (bezogen auf das Volumen des Erythrozytenpellets) Waschpuffer (Stabilisierungsmittel gemäß Beispiel 3 ohne EGTA, ATP und Heparin) aufgenommen und gemischt. Anschließend werden die Zellen wie oben abzentrifugiert und der Waschschritt noch einmal wiederholt. Zur Einstellung der richtigen Konzentration an Erythrozyten werden diese im gleichen Volumen Stabilisierungsmittel gemäß Beispiel 3 resuspendiert und von einer 1:100 Verdünnung im Stabilisierungspuffer die Extinktion bei 578 nm bestimmt. Aus diesem Wert wird die angestrebte Verdünnung berechnet und die Erythrozyten auf den entsprechenden Wert verdünnt. Anschließend werden die suspendierten Erythrozyten portionsweise abgefüllt. Es wurde festgestellt, daß derartig hergestellte Suspensionen bei 2-8° C 2 Jahre gelagert werden können, ohne daß eine relevante Hämolyse oder irgendeine Einbuße in der diagnostischen Verwendbarkeit auftreten (Tabelle 1).

Tabelle 1:

| Lagerungsdauer der Erythrozyten | Hämolyse % | Lysezeit im Komplement test für ein Normalplasma sec |
|---|---|---|
| 0 | 0 | 45 |
| 1 Monat | 0 | 44 |
| 6 Monate | 0 | 44 |
| 12 Monate | 0 | 43 |
| 18 Monate | 1 | 42 |
| 24 Monate | 3 | 45 |

Beispiel 2

Herstellung einer Suspensionslösung

80 mmol Glukose, 150 mmol Natriumchlorid, 10 mmol Kaliumchlorid, 0.5 mmol Magnesiumchlorid, 10 mmol HEPES, 10 mmol EDTA, 1 g Gelatine und 2 mmol ATP werden eingewogen und in 900 ml Aqua dest. gelöst. Dann werden 10 000 Einheiten Heparin sowie 0,05 % Kathon zugegeben. Nach Einstellen des pH-Wertes auf 6,8 wird auf 1 Liter mit Aqua dest. aufgefüllt. Die Lösung wird anschließend unter Druck durch Membranfilter mit einer Porenweite von 0,2 $\mu$m sterilfiltriert.

Beispiel 3

Herstellung einer modifizierten Suspensionslösung

80 mmol Glukose, 150 mmol Natriumchlorid, 10 mmol Kaliumchlorid, 0.5 mmol Magnesiumchlorid, 10 mmol HEPES, 40 mmol $NaH_2PO_4$, 2 mmol EGTA, 1 g Gelatine und 2 mmol ATP werden eingewogen und in 900 ml Aqua dest gelöst. Dann werden 10 000 Einheiten Heparin sowie 0,5 g Kathon zugegeben. Nach Einstellen des pH-Wertes auf 6,8 wird auf 1 Liter mit Aqua dest. aufgefüllt. Die Lösung wird anschließend unter Druck durch Membranfilter mit einer Porenweite von 0,2 $\mu$m sterilfiltriert.

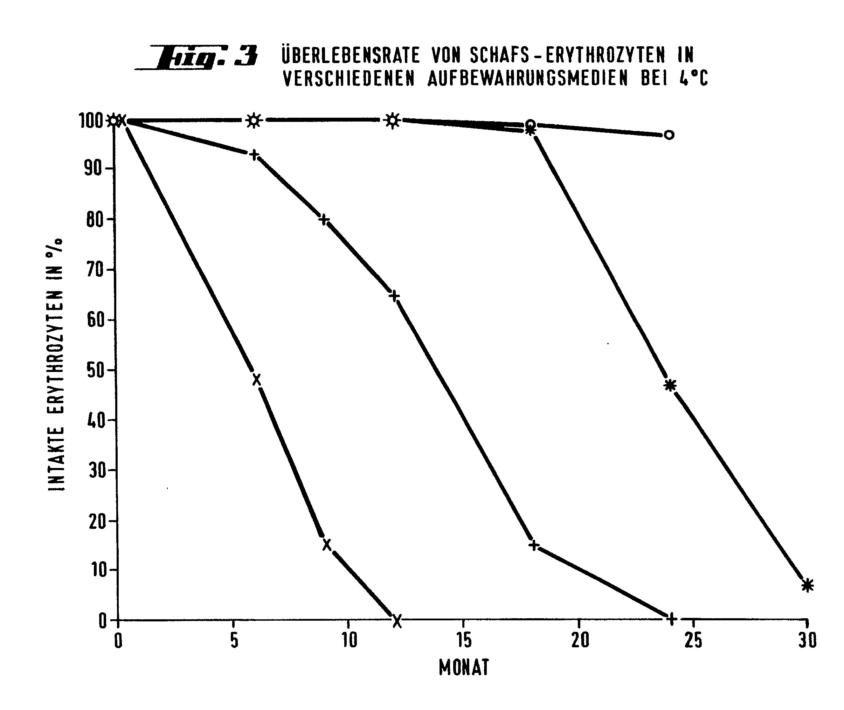

Die Fig. 3 zeigt den zeitlichen Verlauf der Hämolyse über 2 Jahren bei 4° C im Vergleich zu Lösungen nach Stand der Technik ( 80 mmol/l Glukose, 150 mmol/l NaCl, 10 mmol/l HEPES, 10 mmol/l KCl, 0.5 mmol/l Adenin, 0.1 % Gelatine (x-x); nach Stand der Technik und mit Chelator EDTA (+-+); mit der Lösung nach Beispiel 2 (*-*), sowie mit der Lösung nach Beispiel 3 (□-□).

Beispiel 4

Durchführung einer Aktivitätsbestimmung von C4 mittels der Erythrozytensuspension

Fertigstellung des Reagenzes: 0,5 ml der Erythrozytensuspension werden mit 5 ml einer Lösung die Antikörper gegen die Erythrozyten enthält gemischt und auf 37° C erwärmt. Damit ist das Reagenz gebrauchsfertig. Durchführung der Bestimmung: 10 $\mu$l Probe wird mit 100 $\mu$l eines C4 Mangelplasmas versetzt. Dazu wird 1 ml des Reagenzes zupipettiert und die Zeit bestimmt, die durch Lyse der Erythrozyten zu einem Extinktionsabfall bei 578 nm um 0.1 E verstreicht. Der Vergleich der erhaltenen Zeit mit den Werten aus einer vorher analog erstellen Eichkurve ergibt den Gehalt an funktionell aktivem C4.

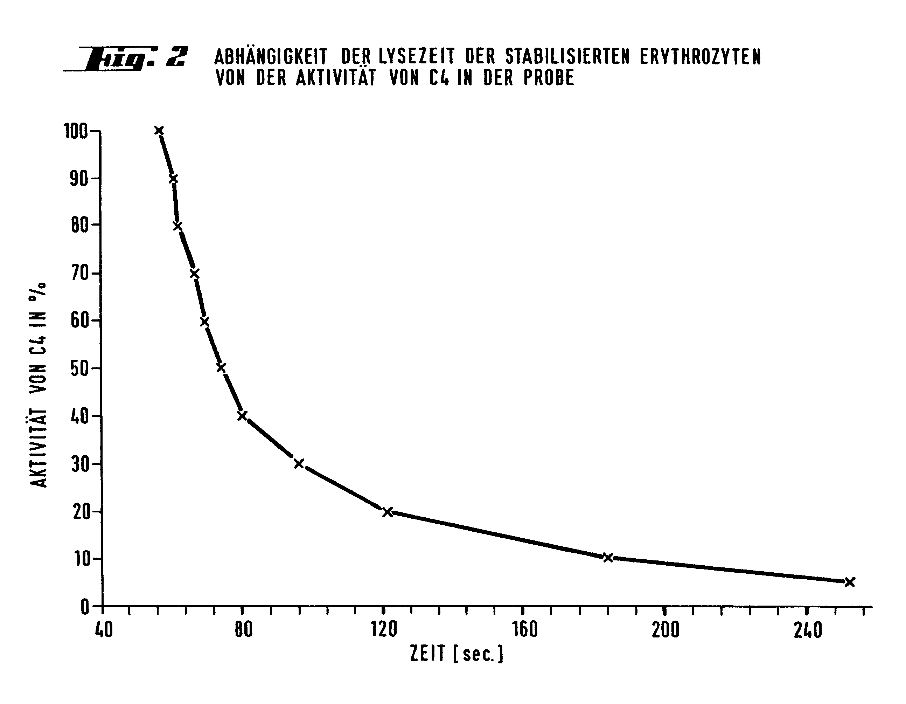

Die Figur 2 zeigt die Abhängigkeit der Lysezeit der stabilisierten Erythrozyten von der Aktivität von C 4 in der Probe.

Beispiel 5:

Durchführung einer Aktivitätsbestimmung vom Gesamtkomplement mittels der Erythrozytensuspension

4

Fertigstellung des Reagenzes: 0,5 ml der Erythrozytensuspension werden mit 5 ml einer Lösung, die Antikörper gegen die Erythrozyten enthält, gemischt und auf 37°C erwärmt. Damit ist das Reagenz gebrauchsfertig. Durchführung der Bestimmung: Zu 100 µl Probe wird 1 ml des Reagenzes zupipettiert und die Zeit bestimmt, die durch Lyse der Erythrozyten zu einem Extinktionsabfall bei 578 nm um 0.1 E verstreicht. Der Vergleich der erhaltenen Zeit mit den Werten aus einer vorher analog erstellen Eichkurve aus Verdünnungen eines Poolplasmas mit isotonischer Kochsalzlösung ergibt die Komplementaktivität der Probe.

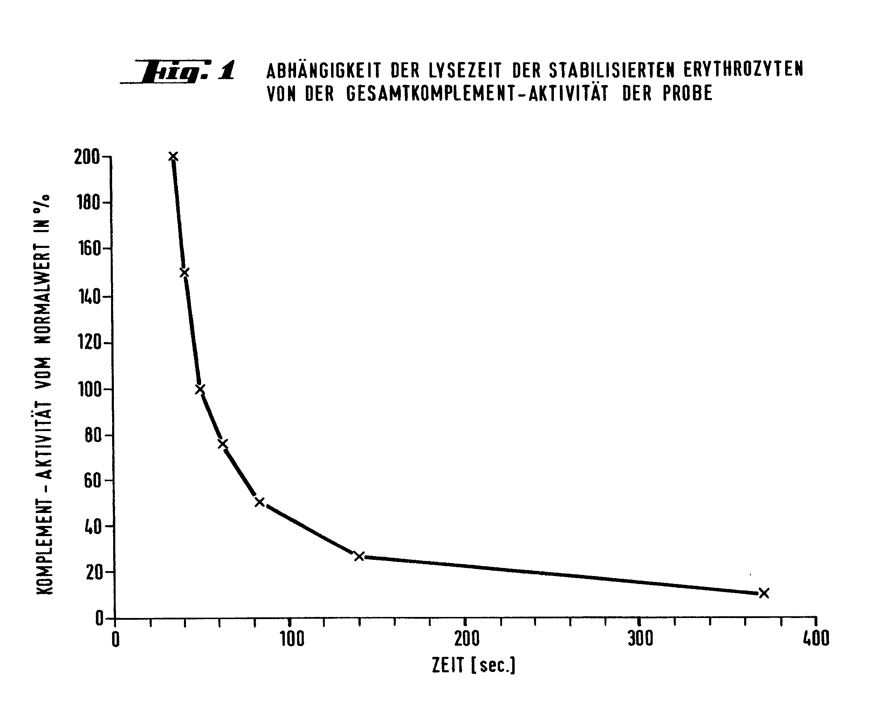

Die Figur 1 zeigt die Abhängigkeit der Lysezeit der stabilisierten Erythrozyten von der Gesamtkomplement-Aktivität der Probe.

## Ansprüche

1. Mittel zum Suspendieren, Konservieren und Lagern von biologisch aktiven Zellen oder Zellbestandteilen, insbesondere Erythrozyten, dadurch gekennzeichnet, daß es einen Chelatbildner für mehrwertige Metallionen in einer wässrigen, gepufferten Lösung enthält.

2. Mittel gemäß Anspruch 1, dadurch gekennzeichnet, daß es zusätzlich entweder ATP oder ein wasserlösliches Phosphat oder beides enthält.

3. Mittel gemäß Anspruch 1 bis 2, dadurch gekennzeichnet, daß es außerdem mindestens eine der folgenden Substanzen enthält: Heparin, Kaliumionen, Gelatine, chemisch modifizierte Gelatine und HEPES.

4. Mittel gemäß mindestens einem der Ansprüche 1 oder 3, dadurch gekennzeichnet, daß der Chelatbildner EDTA, EGTA oder Oxalessigsäure ist.

5. Mittel gemäß mindestens einem der Ansprüche 1 bis 4, dadurch gekennzeichnet, daß die verwendungsfähige Suspension von biologisch aktiven Zellen oder Zellbestandteilen pro Liter 0 - 100 mmol Glukose oder Fruktose, 20 - 200 mmol Natriumchlorid, 0 - 50 mmol Kaliumchlorid, 0 - 100 mmol Magnesiumchlorid, 2 - 100 mmol HEPES, 0 - 100 mmol Na-Phosphat, 0.1 - 50 mmol des Chelatbildners, 0 - 20 000 Einheiten Heparin, 0 -5 g Gelatine oder Polygelin, 0 - 50 mmol ATP und 0 -1 g Kathon oder Chloramphenicol enthält und der pH zwischen 5 und 9, bevorzugterweise bei pH 6.5 bis 7.5 liegt.

6. Verwendung eines Mittels gemäß mindestens einem der Ansprüche 1 bis 5 als Bestandteil eines Diagnostikums zur Bestimmung der Aktivität des Komplementsystems.

7. Verwendung eines Mittels gemäß mindestens einem der Ansprüche 1 bis 5 als Bestandteil eines Diagnostikums zur Bestimmung der Aktivität von Einzelfaktoren oder Inhibitoren des Komplementsystems.

8. Verfahren zum Suspendieren, Konservieren und Lagern von biologisch aktiven Zellen oder Zellbestandteilen, insbesondere Erythrozyten, dadurch gekennzeichnet, daß ein Mittel gemäß einem der Ansprüche 1 bis 3 verwendet wird.

Patentansprüche für folgenden Vertragsstaat: ES

1. Verfahren zur Herstellung eines Mittels zum Suspendieren, Konservieren und Lagern von biologisch aktiven Zellen oder Zellbestandteilen, insbesondere Erythrozyten, dadurch gekennzeichnet, das ein Chelatbildner für mehrwertige Metallionen in einer wässrigen, gepufferten Lösung gelöst wird.

2. Verfahren gemäß Anspruch 1, dadurch gekennzeichnet, das das Mittel zusätzlich entweder ATP oder ein wasserlösliches Phosphat oder beides enthält.

3. Verfahren gemäß Anspruch 1 bis 2, dadurch gekennzeichnet, das das Mittel auserdem mindestens eine der folgenden Substanzen enthält: Heparin, Kaliumionen, Gelatine, chemisch modifizierte Gelatine und HEPES.

4. Verfahren gemäs mindestens einem der Ansprüche 1 oder 3, dadurch gekennzeichnet, das der Chelatbildner EDTA, EGTA oder Oxalessigsäure ist.

5. Verfahren gemäs mindestens einem der Ansprüche 1 bis 4, dadurch gekennzeichnet, das die verwendungsfähige Suspension von biologisch aktiven Zellen oder Zellbestandteilen pro Liter 0 - 100 mmol Glukose oder Fruktose, 20 - 200 mmol Natriumchlorid, 0 - 50 mmol Kaliumchlorid, 0 - 100 mmol Magnesiumchlorid, 2 - 100 mmol HEPES, 0 - 100 mmol Na-Phosphat, 0.1 - 50 mmol des Chelatbildners, 0 - 20 000 Einheiten Heparin, 0 - 5 g Gelatine oder Polygelin, 0 -50 mmol ATP und 0 - 1 g Kathon oder Chloramphenicol enthält und der pH zwischen 5 und 9, bevorzugterweise bei pH 6.5 bis 7.5 liegt.

6. Verwendung eines Mittels gemäß einem der Ansprüche 1 bis als Bestandteil eines Diagnostikums zur Bestimmung der Aktivität des Komplementsystems.

7. Verwendung eines Mittels gemäß mindestens einem der Ansprüche 1 bis 5 als Bestandteil eines Diagnostikums zur Bestimmung der Aktivität von Einzelfaktoren oder Inhibitoren des Komplementsystems.

8. Verfahren zum Suspendieren, Konservieren und Lagern von biologisch aktiven Zellen oder Zellbestandteilen, insbesondere Erythrozyten, dadurch gekennzeichnet, das ein Mittel gemäß einem der Ansprüche 1 bis 3 verwendet wird.

EP 0 431 385 A1

**Fig. 1** ABHÄNGIGKEIT DER LYSEZEIT DER STABILISIERTEN ERYTHROZYTEN VON DER GESAMTKOMPLEMENT-AKTIVITÄT DER PROBE

**Fig. 2** ABHÄNGIGKEIT DER LYSEZEIT DER STABILISIERTEN ERYTHROZYTEN VON DER AKTIVITÄT VON C4 IN DER PROBE

EP 0 431 385 A1

**Fig. 3** ÜBERLEBENSRATE VON SCHAFS-ERYTHROZYTEN IN VERSCHIEDENEN AUFBEWAHRUNGSMEDIEN BEI 4°C

INTAKTE ERYTHROZYTEN IN %

MONAT

EP 0 431 385 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 236 949   (TAKEDA CHEMICAL INDUSTRIES LTD) <br> * Seite 2, Zeilen 8-26; Beispiel 1, Tabelle 1; Patentansprüche * <br><br> – – – | 1-5,8 | A 01 N <br> 1/02 <br> G 01 N 33/68 |
| X | EP-A-0 053 046   (G.A. ROCK) <br> * Seite 7, Zeilen 16-28; Patentansprüche * <br> – – – | 1-5,8 | |
| X | DE-A-2 710 674   (RESEARCH CORP., New York) <br> * Patentansprüche; Seite 2, Zeile 4 - Seite 3, Zeile 18 * <br> – – – | 1-5,8 | |
| X | CHEMICAL ABSTRACTS, Band 103, Nr. 19, 11. November 1985, Seite 363, Zusammenfassung Nr. 156762f, Columbus, Ohio, US; H. NIELSEN: "Influence of five different anticoagulants on human blood monocyte isolation and functional activities", <br> & ACTA PATHOL. MICROBIOL. IMMUNOL. SCAND., SECT. C 1985, 93C(2), 49-52 <br> * Zusammenfassung * <br><br> – – – | 1-5,8 | |
| X | CHEMICAL ABSTRACTS, Band 88, Nr. 25, 19. Juni 1978, Seite 450, Zusammenfassung Nr, 186980q, Columbus, Ohio, US; S. HEPTINSTALL et al.: "The influence of anticoagulants and extracellular divalent cations on blood platelet behavior", <br> & J. PHYSIOL. (London) 1978, 276, 25P-26P <br> * Zusammenfassung * <br><br> – – – | 1-5,8 | RECHERCHIERTE SACHGEBIETE (Int. Cl.5) <br><br> A 01 N <br> A 61 K |
| X | CHEMICAL ABSTRACTS, Band 83, Nr. 1, 7. Juli 1975, Seiten 526,527, Zusammenfassung Nr. 6072k, Columbus, Ohio, US; D. BEIER et al.: "Comparison of various combinations of anticoagulants and stabilizers for preserving blood samples for leucosis diagnosis. II. Tests with a new combination", <br> & ARCH. EXP. VETERINAERMED. 1975, 29(1), 147-51 <br> * Zusammenfassung * <br><br> – – – <br><br> –/– | 1-8 | |

Der vorliegende Recherchenbericht wurde für alle Patentansprüche erstellt

| Recherchenort | Abschlußdatum der Recherche | Prüfer |
|---|---|---|
| Den Haag | 14 März 91 | DONOVAN T.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

------------------------------------------------

& : Mitglied der gleichen Patentfamilie, übereinstimmendes Dokument

**Europäisches
Patentamt**

# EUROPÄISCHER
# RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 420 461 (R.P. RECKEL et al.)<br>* Patentansprüche *<br>– – – | 6,7 | |
| A | DERWENT CENTRAL PATENTS INDEX, SCHNITT C, Woche 30, Zusammenfassung Nr. 87-208884 [30], Derwent Publications Ltd, London, GB;<br>& JP-A-62 134 561<br>– – – – – | 6,7 | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| Den Haag | 14 März 91 | DONOVAN T.M. |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
nach dem Anmeldedatum veröffentlicht worden ist
D : in der Anmeldung angeführtes Dokument
L : aus anderen Gründen angeführtes Dokument
......................................................................
& : Mitglied der gleichen Patentfamilie,
übereinstimmendes Dokument